# EUROPEAN PATENT SPECIFICATION

(11) **EP 4 205 700 B1**
(45) Date of publication and mention of the grant of the patent: **25.06.2025**
(21) Application number: 21861939.3
(22) Date of filing: 10.08.2021
(51) Int. Cl.: A61F 2/06, A61L 27/50, A61L 27/54, A61L 27/56, A61F 2/00, A61F 2/82

(54) **POROUS MULTILAYER TUBULAR STRUCTURE HAVING PHYSIOLOGICALLY ACTIVE SUBSTANCES CONTAINED IN PORES**
PORÖSE MEHRSCHICHTIGE RÖHRENFÖRMIGE STRUKTUR MIT IN DEN POREN ENTHALTENEN PHYSIOLOGISCH AKTIVEN SUBSTANZEN
STRUCTURE TUBULAIRE MULTICOUCHE POREUSE AYANT DES SUBSTANCES PHYSIOLOGIQUEMENT ACTIVES CONTENUES DANS DES PORES

(30) Priority: 28.08.2020 KR 20200108913
(43) Date of publication of application: 05.07.2023
(73) Proprietor: Kim, Dae Joong, Gyeonggi-do 13558 (KR)
(72) Inventor: BAEK, In Su, Anyang-si Gyeonggi-do 13979 (KR)
(74) Representative: dompatent von Kreisler Selting Werner - Partnerschaft von Patent- und Rechtsanwälten mbB
(86) International application number: PCT/KR2021/010543
(87) International publication number: WO 2022/045642

(56) References cited:
- WO-A2-2004/050140
- WO-A2-2006/020742
- JP-A- 2007 190 369
- JP-A- 2009 531 137
- KR-A- 20150 042 187
- US-A1- 2009 029 077
- US-B2- 7 294 409

## Description

### Technical Field

The present disclosure relates to a tubular structure.

### Background Art

There are cases in which a tubular structure having a lumen is inserted into the human body for the purpose of treatment or prevention. Representative examples include a vascular conduit or a vascular access (hemodialysis access) for dialysis.

In general, vascular conduits are artificial blood vessels that may be used to replace living blood vessels when there is a vascular disease that is difficult to cure by surgery or pharmacological treatment. However, since the vascular conduit is not a natural structure of the human body, various problems may arise. Representative problems are stenosis at a connection site between a vascular conduit and blood vessels of the human body and surrounding sites, thrombosis with stenosis, and an inflammatory reaction at the site of the vascular conduit. In order to solve these problems, studies have been conducted to prevent vascular stenosis and inflammation by forming a physiologically active substance layer or a drug layer in a vascular conduit. US 7 294 409 B2 discloses porous multilayer polymeric tubular structures comprising a drug in the pores.

However, since the effect of the drug on the inside or outside of the vascular conduit may vary depending on the type of drug layer, it is necessary to control this variation by a preferred method. In addition, since the properties of the vascular conduit may vary depending on the method of forming the drug layer, the development of a new technology considering this variation is required.

### Disclosure

### Technical Problem

The technical spirit of the present disclosure is intended to solve the above-described problems, and an object of the technical spirit of the present disclosure is to provide a technology for a tubular structure that may be appropriately controlled to show different effects on the inside and outside of the vascular conduit depending on the type of drug.

Another object of the technical spirit of the present disclosure is to provide a technology for a tubular structure that is capable of appropriately controlling the drug release rate.

Objects to be achieved by the present invention are not limited to the above-mentioned objects, and other objects not mentioned herein will be clearly understood by those skilled in the art from the following description.

### Technical Solution

To achieve the above objects, it is provided a porous multi-layered tubular structure according to claim 1.

In addition, the amount of the physiologically active substance contained in at least one of the layers may be different from the amount of the physiologically active substance contained in the other layer.

In addition, at least one of the layers may be formed to be different from the other layer in terms of the shape of the pores, the size of the pores, the arrangement of the pores, or the number of pores per unit area of the layer, so that the content of the physiologically active substance in the pores of each of the layers and the release rate of the physiologically active substance may be set differently for each of the layers.

In addition, the physiologically active substance may be a substance that inhibits cell proliferation or cell migration.

In addition, the physiologically active substance may be at least one selected from the group consisting of paclitaxel, rapamycin, and everolimus.

In addition, the content of the physiologically active substance in each of the layers may decrease in the direction from the innermost layer to the outermost layer of the tubular structure.

In addition, the outermost layer of the tubular structure may not contain the physiologically active substance.

In addition, when the physiologically active substance is paclitaxel, the content of the physiologically active substance per area of the innermost layer of the tubular structure may be 0.25 to 1.5 µg/mm².

In addition, when the physiologically active substance is paclitaxel, the content of the physiologically active substance per area of the layer positioned next to the innermost layer of the tubular structure may be 0.25 to 0.75 µg/mm².

In addition, the outermost layer of the tubular structure may not contain the physiologically active substance, or when the physiologically active substance is paclitaxel, the content of the physiologically active substance per area of the outermost layer may be 0.25 µg/mm². or less.

In addition, the outermost layer of the tubular structure may have a porosity of less than 25 µm as measured according to International Standardization Organization (ISO) standard 7198:1998.

The material of each of the layers is at least any one selected from the group consisting of expanded polytetrafluoroethylene, polytetrafluoroethylene, and polyurethane.

In addition, the content of the physiologically active substance in each of the layers may decrease in the direction from the outermost layer to the innermost layer of the tubular structure.

In addition, the tubular structure is formed of three or more layers, and the porosity of a layer positioned next to the outermost layer of the tubular structure is smaller than the porosities of the other layers, as measured according to International Standardization Organization (ISO) standard 7198:1998.

In addition, the porosity of a layer positioned next to the outermost layer of the tubular structure may be less than 25 µm as measured according to International Standardization Organization (ISO) standard 7198:1998.

In addition, all of the plurality of layers may be formed of the same material.

In addition, when the tubular structure is formed of three layers, the porosity of a layer positioned between the outermost layer and the innermost layer of the tubular structure is smaller than the porosities of the other layers.

### Advantageous Effects

As described above, according to various embodiments of the present invention, the content of the physiologically active substance may be set differently for each of the layers, and thus the release rate of the physiologically active substance *in vivo* may be appropriately controlled.

In addition, according to various embodiments of the present invention, the content of the physiologically active substance in each layer may decrease in the direction from the innermost layer to the outermost layer of the tubular structure, and the outermost layer may not contain the physiologically active substance, and thus it is possible to prevent the physiologically active substance from inhibiting proliferation of myofibroblasts outside of the vascular conduit.

In addition, according to various embodiments of the present invention, the size, number, shape, arrangement, etc. of pores may be different between layers, and thus the content of the physiologically active substance in the pores may be controlled differently for each of the layers. Thereby, it is possible to prevent a physiologically active substance that inhibits cell proliferation or cell migration from permeating from the inner circumferential surface to the outer circumferential surface of the tubular structure and reaching the outer circumferential surface.

In addition, according to the implementation, the content of the physiologically active substance in each layer may be controlled so that it decreases in the direction from the outermost layer to the innermost layer of the tubular structure and the innermost layer does not contain the physiologically active substance. Therefore, it is possible to selectively apply the physiologically active substance only to the outside of the tubular structure in consideration of the efficacy and type of physiologically active substance.

Effects according to various embodiments of the present invention are not limited to the above-mentioned effects, and other effects not mentioned herein will be clearly understood by those skilled in the art from the following description.

### Description of Drawings

FIG. 1 schematically shows a tubular structure according to a first embodiment, not according to the claims;
FIG. 2 schematically shows a tubular structure according to a second embodiment, not according to the claims;
FIG. 3 schematically shows a tubular structure according to a third embodiment, according to the present invention;
FIG. 4 schematically shows a tubular structure according to a fourth embodiment, according to the present invention;
FIG. 5 schematically shows a tubular structure according to a fifth embodiment, according to the present invention;
FIG. 6 schematically shows a tubular structure according to a sixth embodiment, according to the present invention; and
FIG. 7 schematically shows a tubular structure according to a seventh embodiment, according to the present invention.

### Mode for Invention

Preferred embodiments of the present invention will be described in more detail with reference to the accompanying drawings. The descriptions of already known technical features will be omitted or simplified for the sake of brevity.

It should be noted that references to "an" or "one" embodiment of the invention in this specification are not necessarily directed to the same embodiment, and they imply at least one.

In the following embodiments, terms such as "first" and "second" are not used in a limited sense, but are used for the purpose of distinguishing one component from another component.

In the following examples, singular expressions include plural expressions unless the context clearly indicates otherwise.

In the following embodiments, terms such as "include" and "have" are intended to denote the presence of mentioned features or components, but do not exclude the probability of presence or addition of one or more other features or components.

In the following embodiments, when a part, such as a layer, region, component, or the like, is referred to as being "on" or "above" another part, it not only refers to a case where the part may be directly above the other part, but also a case where a third part exists therebetween.

Sizes, heights, thicknesses, and the like in the drawings referred to when describing the embodiments of the present disclosure may be intentionally exaggerated and expressed for convenience of description and to facilitate understanding, and are not enlarged or shrunk according to any ratio. In addition, some elements illustrated in the drawings may be intentionally expressed as being smaller, and the other elements may be intentionally expressed as being larger.

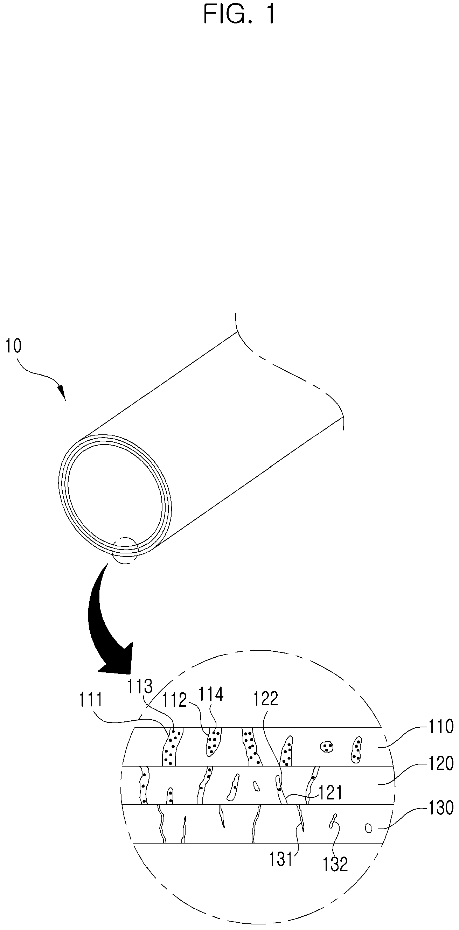

FIG. 1 schematically shows a tubular structure according to a first embodiment, not according to the claims. Referring to FIG. 1, the tubular structure 10 according to the first embodiment is a structure having a lumen and may be formed by stacking three layers. That is, a first layer 110 is the innermost layer of the tubular structure 10, and a second layer 120 is attached to the outer circumferential surface of the first layer 110. In addition, a third layer 130 is attached to the outer circumferential surface of the second layer 120 and is the outermost layer of the tubular structure 10.

In the first embodiment, a plurality of pores may be formed in each of the layers constituting the tubular structure 10. According to the first embodiment, a plurality of pores may be formed in the first layer 110, the second layer 120, and the third layer 130. In addition, a physiologically active substance may be contained in at least one pore in at least one of the layers.

In the first embodiment, the physiologically active substance refers to a substance that inhibits cell proliferation or cell migration. For example, the physiologically active substance may be at least one selected from the group consisting of paclitaxel, rapamycin, and everolimus.

According to the first embodiment, the porosity of the pores 111 and 112 formed in the first layer 110 among the plurality of layers may be different from the porosity of the pores 121 formed in the second layer 120. That is, by making the porosity different between the layers, it is possible to control the content of the physiologically active substance in the pores 111 and 112 of the first layer 110 so as to be different from the content of the physiologically active substance in the pores 121 of the second layer 120. As used herein, the term "porosity" means a microscopic porosity value measured according to International Organization Standardization (ISO) standard 7198:1998

As a specific example, in the first embodiment, the porosity of the pores 111 and 112 formed in the first layer 110 among the three layers may be larger than that of the pores 121 formed in the second layer 120, and the porosity of the pores 131 and 132 formed in the third layer 130 may be smaller than that of the pores 121 formed in the second layer 120. Thus, the content of the physiologically active substance in each pore decreases in the direction from the first layer 110 to the third layer 130. In addition, the porosity of each layer may be controlled so that the pores 131 and 132 of the third layer 130 do not contain the physiologically active substance, unlike the first layer 110 or the second layer 120.

According to the first embodiment, the shape, size, or arrangement of the pores 111 and 112 formed in the first layer 110 among the plurality of layers constituting the tubular structure 10, or the number of the pores 111 and 112 per unit area of the first layer, may be different from the shape, size, or arrangement of the pores 121 formed in the second layer 120, or the number of pores 121 per unit area of the second layer. That is, when the shape, size, arrangement of pores or the number of pores per unit area of each layer is set differently for each of the layers, the content of the physiologically active substance in the pores 111 and 112 of the first layer 110 may be different from the content of the physiologically active substance in the pores 121 of the second layer 120.

As a specific example, when 30 pores are formed in the first layer 110, 20 pores are formed in the second layer 120, and 10 pores are formed in the third layer 130, the content of the physiologically active substance in the pores will be different between the layers. In addition, at least any one of the number, shape, and arrangement of the pores may be controlled so that the physiologically active substance is not contained in the pores 131 and 132 of the third layer 130.

According to the first embodiment, the content of the physiologically active substance per area of the first layer 110, which is the innermost layer of the tubular structure 10, is 0.25 to 1.5 µg/mm². regardless of the thickness of the layer, when the physiologically active substance is paclitaxel. As a specific example, the content of the physiologically active substance per area of the first layer 110 may be 0.25 µg/mm², 0.35 µg/mm², 0.45 µg/mm², 0.55 µg/mm², 0.65 µg/mm², 0.75 µg/mm², 0.85 µg/mm², 0.95 µg/mm², 1.05 µg/mm², 1.15 µg/mm², 1.25 µg/mm², 1.35 µg/mm², 1.45 µg/mm², or 1.5 µg/mm². regardless of the thickness of the layer, when the physiologically active substance is paclitaxel. In addition, the content of the physiologically active substance per area of the first layer 110 may range from a value equal to or greater than any one of the above values to a value equal to or smaller than any one of the above values.

For example, the content of the physiologically active substance per area of the first layer 110 may be in the range of 0.25 µg/mm². to 1.5 µg/mm², 0.35 µg/mm². to 1.45 µg/mm², 0.55 µg/mm². to 1.35 µg/mm², 0.65 µg/mm². to 1.25 µg/mm², 0.75 µg/mm². to 1.15 µg/mm², or 0.85 µg/mm². to 1.05 µg/mm². regardless of the thickness of the layer, when the physiologically active substance is paclitaxel.

According to the first embodiment, the content of the physiologically active substance per area of the second layer 120 stacked on the outer circumferential surface of the first layer 110 may be 0.25 to 0.75 µg/mm². regardless of the thickness of the layer, when the physiologically active substance is paclitaxel. As a specific example, the content of the physiologically active substance per area of the second layer 120 may be 0.25 µg/mm², 0.35 µg/mm², 0.45 µg/mm², 0.55 µg/mm², 0.65 µg/mm². or 0.75 µg/mm². regardless of the thickness of the layer, when the physiologically active substance is paclitaxel. In addition, the content of the physiologically active substance per area of the second layer 120 may range from a value equal to or greater than any one of the above values to a value equal to or smaller than any one of the above values.

For example, the content of the physiologically active substance per area of the second layer 120 may be in the range of 0.25 µg/mm². to 0.75 µg/mm², 0.35 µg/mm². to 0.65 µg/mm², or 0.45 µg/mm². to 0.55 µg/mm². regardless of the thickness of the layer, when the physiologically active substance is paclitaxel.

Meanwhile, according to the implementation, the third layer 130, which is the outermost layer of the tubular structure 10, may contain the physiologically active substance in a smaller amount than the first layer 110 or the second layer 120 (for example, when the physiologically active substance is paclitaxel, the content of the physiologically active substance in the third layer may be 0.01 to 0.25 µg/mm². regardless of the thickness of the layer).

According to the first embodiment, the third layer 130, which is the outermost layer of the tubular structure 10, may be formed so that the porosity of the pores 131 and 132 is smaller than those of the other layers. In addition, the third layer 130 may be formed so that the number of pores 131 and 132 is smaller than those in the other layers. Thereby, the third layer 130 may not contain the physiologically active substance at all or may contain the physiologically active substance in a smaller amount than the other layers.

The tubular structure 10 according to the first embodiment has a plurality of layers, wherein the average size of pores formed in each of the layers may be 0.1 µm to less than 25 µm as measured according to International Standardization Organization (ISO) standard 7198.

In the first embodiment, the material of each of the layers constituting the tubular structure 10 may be at least one selected from the group consisting of expanded polytetrafluoroethylene, polytetrafluoroethylene, and polyurethane. Meanwhile, according to the implementation, all of the plurality of layers may be formed of the same material. In one embodiment, when all of the layers constituting the multi-layered tubular structure are formed of the same material, the elasticity and ductility of each layer may be equal between the layers, and it is possible to prevent the adjacent layers from falling off or cracking when the tubular structure is bent by an external force.

The tubular structure 10 according to the first embodiment may be manufactured by spraying a mixed solution of a polar solvent, a non-polar solvent and a physiologically active substance toward the inner circumferential surface of the first layer 110 in a state in which the first layer 110, the second layer 120 and the third layer 130 are stacked in advance. That is, it may be manufactured by positioning a spraying device inside the tubular structure 10 and spraying the solution toward the inner circumferential surface of the first layer 110.

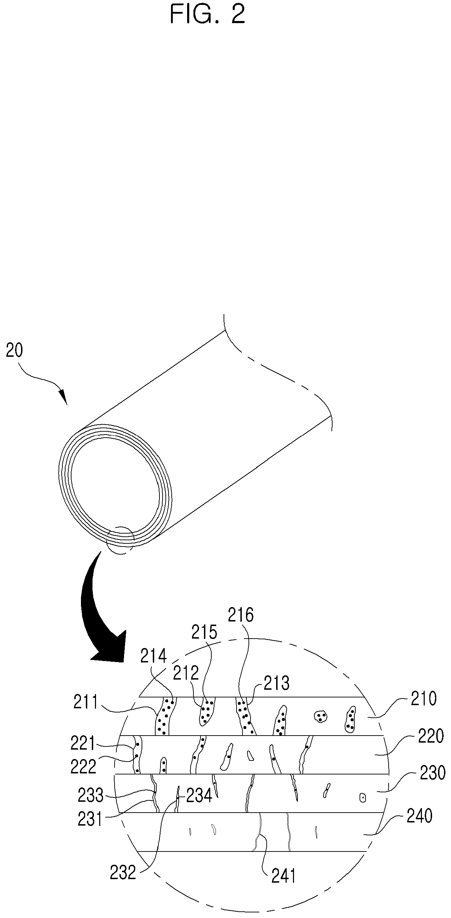

FIG. 2 schematically shows a tubular structure according to a second embodiment, not according to the claims. Referring to FIG. 2, the tubular structure 20 according to the second embodiment may be formed by stacking four layers. That is, a first layer 210 is the innermost layer of the tubular structure 20. A second layer 220 is attached to the outer circumferential surface of the first layer 210, and a third layer 230 is attached to the outer circumferential surface of the second layer 220. A fourth layer 240 is attached to the outer circumferential surface of the third layer 230 and is the outermost layer of the tubular structure 20.

In the second embodiment, a plurality of pores may be formed in each of the layers constituting the tubular structure 20. According to the second embodiment, a plurality of pores may be formed in the first layer 210, the second layer 220, the third layer 230 and the fourth layer 240. In addition, a physiologically active substance may be contained in at least one pore in at least one of the layers.

In the second embodiment, the physiologically active substance may be applied in the same way as in the first embodiment. According to the second embodiment, the porosity of the pores 211, 212, and 213 formed in the first layer 210 among the plurality of layers may be different from the porosity of the pores 221 formed in the second layer 220. That is, by making the porosity of the pores different between the layers, it is possible to control the content of the physiologically active substance in the pores 211, 212 and 213 of the first layer 210 so as to be different from the content of the physiologically active substance in the pores 221 of the second layer 220.

As a specific example, in the second embodiment, the porosity of the pores 211, 212 and 213 formed in the first layer 210 among the four layers may be larger than the porosity of the pores 221 formed in the second layer 220, the porosity of the pores 221 formed in the second layer 220 may be larger than that of the pores 231 and 232 formed in the third layer 230, and the porosity of the pores 231 and 232 formed in the third layer 230 may be larger than that of the pores 241 formed in the fourth layer 240. Thus, the content of the physiologically active substance in each pore decreases in the direction from the first layer 210 toward the fourth layer 240. In addition, it is possible to control the porosity of the pores so that the pores 241 of the fourth layer 240 do not contain the physiologically active substance, unlike the first layer 210, the second layer 220 and the third layer 230.

According to the second embodiment, the shape, size, or arrangement of the pores 211, 212 and 213 formed in the first layer 210 among the plurality of layers constituting the tubular structure 20, or the number of the pores 211, 212 and 213 per unit area of the first layer, may be different from the shape, size, or arrangement of the pores 221 formed in the second layer 220, or the number of pores 221 per unit area of the second layer. That is, when the shape, size, arrangement of pores or the number of pores per unit area of each layer is set differently for each of the layers, the content of the physiologically active substance in the pores 211, 212 and 213 of the first layer 210 may be different from the content of the physiologically active substance in the pores 221 of the second layer 220.

As a specific example, when 30 pores are formed in the first layer 210, 20 pores are formed in the second layer 220, 10 pores are formed in the third layer 230, and 5 pores are formed in the fourth layer 240, the content of the physiologically active substance in the pores will be different between the layers. In addition, at least any one of the number, shape, and arrangement of the pores may be controlled so that the physiologically active substance is not contained in the pores 241 of the fourth layer 240.

According to the second embodiment, the content of the physiologically active substance per area of the first layer 210, which is the innermost layer of the tubular structure 20, may be 0.25 to 1.5 µg/mm². As a specific example, the content of the physiologically active substance per area of the first layer 210 may be 0.25 µg/mm², 0.35 µg/mm², 0.45 µg/mm², 0.55 µg/mm², 0.65 µg/mm², 0.75 µg/mm², 0.85 µg/mm², 0.95 µg/mm², 1.05 µg/mm², 1.15 µg/mm², 1.25 µg/mm², 1.35 µg/mm², 1.45 µg/mm², or 1.5 µg/mm². regardless of the thickness of the layer, when the physiologically active substance is paclitaxel. In addition, the content of the physiologically active substance per area of the first layer 210 may range from a value equal to or greater than any one of the above values to a value equal to or smaller than any one of the above values.

For example, the content of the physiologically active substance per area of the first layer 210 may be in the range of 0.25 µg/mm². to 1.5 µg/mm², 0.35 µg/mm². to 1.45 µg/mm², 0.55 µg/mm². to 1.35 µg/mm², 0.65 µg/mm². to 1.25 µg/mm², 0.75 µg/mm². to 1.15 µg/mm², or 0.85 µg/mm². to 1.05 µg/mm². regardless of the thickness of the layer, when the physiologically active substance is paclitaxel.

According to the second embodiment, the content of the physiologically active substance per area of the second layer 220 attached to the outer circumferential surface of the first layer 210 may be 0.25 to 0.75 µg/mm². regardless of the thickness of the layer, when the physiologically active substance is paclitaxel. As a specific example, the content of the physiologically active substance per area of the second layer 220 may be 0.25 µg/mm², 0.35 µg/mm², 0.45 µg/mm², 0.55 µg/mm², 0.65 µg/mm². or 0.75 µg/mm². regardless of the thickness of the layer, when the physiologically active substance is paclitaxel. In addition, the content of the physiologically active substance per area of the second layer 220 may range from a value equal to or greater than any one of the above values to a value equal to or smaller than any one of the above values.

For example, the content of the physiologically active substance per area of the second layer 220 may be in the range of 0.25 µg/mm². to 0.75 µg/mm², 0.35 µg/mm². to 0.65 µg/mm², or 0.45 µg/mm². to 0.55 µg/mm². regardless of the thickness of the layer, when the physiologically active substance is paclitaxel.

Meanwhile, according to the implementation, the fourth layer 240, which is the outermost layer of the tubular structure 20, may contain the physiologically active substance in a smaller amount than the other layers (for example, when the physiologically active substance is paclitaxel, the content of the physiologically active substance in the fourth layer may be 0.01 to 0.25 µg/mm². regardless of the thickness of the layer).

According to the second embodiment, the fourth layer 240, which is the outermost layer of the tubular structure 20, may be formed so that the porosity of the pores 241 is smaller than those of the other layers. In addition, the fourth layer 240 may be formed so that the number of the pores 241 is smaller than those in the other layers. Thereby, the fourth layer 240 may not contain the physiologically active substance at all or may contain the physiologically active substance in a smaller amount than the other layers.

The tubular structure 20 according to the second embodiment has a plurality of layers, wherein the average size of pores formed in each of the layers may be 0.1 µm to less than 25 µm as measured according to International Standardization Organization (ISO) standard 7198.

In the second embodiment, the material of each of the layers constituting the tubular structure 20 may be as described above with respect to the first embodiment.

The tubular structure 20 according to the second embodiment may be manufactured by spraying a mixed solution of a polar solvent, a non-polar solvent and a physiologically active substance toward the inner circumferential surface of the first layer 210 in a state in which the first layer 210, the second layer 220, the third layer 230 and the fourth layer 240 are stacked in advance. That is, it may be manufactured by positioning a spraying device inside the tubular structure 10 and spraying the solution toward the inner circumferential surface of the first layer 110.

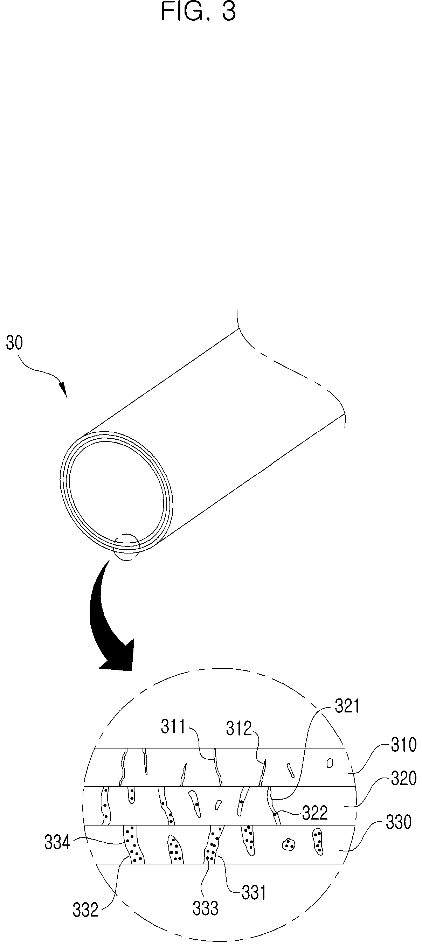

FIG. 3 schematically shows a tubular structure according to a third embodiment, according to the present invention. Referring to FIG. 3, the tubular structure 30 according to the third embodiment may be formed by stacking three layers. That is, a first layer 310 is the innermost layer of the tubular structure 30. A second layer 320 is attached to the outer circumferential surface of the first layer 310, and a third layer 330 is attached to the outer circumferential surface of the second layer 320. The third layer 330 is the outermost layer of the tubular structure 30.

In the third embodiment, a plurality of pores may be formed in each of the layers constituting the tubular structure 30. According to the third embodiment, a plurality of pores may be formed in the first layer 310, the second layer 320, and the third layer 330. In addition, a physiologically active substance may be contained in at least one pore in at least one of the layers.

In the third embodiment, the physiologically active substance may be of a type different from that in the first embodiment. For example, the physiologically active substance according to the third embodiment may be an anti-inflammatory agent. In the case in which it is preferable in terms of the efficacy of the physiologically active substance that the physiologically active substance be contained in an outer layer of the tubular structure 30 rather than contained in an inner layer of the tubular structure 30, control may be made so that the physiologically active substance is selectively applied only to the outer layer without existing in the inner layer of the tubular structure 30.

According to the third embodiment, the porosity of the pores 311 and 312 formed in the first layer 310 among the plurality of layers may be different from the porosity of the pores 321 formed in the second layer 320. That is, by making the porosity of the pores different between the layers, it is possible to control the content of the physiologically active substance in the pores 311 and 312 of the first layer 310 so as to be different from the content of the physiologically active substance in the pores 321 of the second layer 320.

As a specific example, in the third embodiment, the porosity of the pores 331 and 332 formed in the third layer 330 among the three layers is larger than that of the pores 321 formed in the second layer 320, and the porosity of the pores 321 formed in the second layer 320 may be larger than that of the pores 311 and 312 in the first layer 310. Thus, the content of the physiologically active substance in the pores of each layer decreases in the direction from the third layer 330, which is the outermost layer of the tubular structure 30, toward the first layer 310 which is the innermost layer. In addition, the porosity of the pores may be controlled so that the pores 311 and 312 of the first layer 310 do not contain the physiologically active substance, unlike the second layer 320 or the third layer 330.

According to the third embodiment, the shape, size, or arrangement of the pores 311 and 312 formed in the first layer 310 among the plurality of layers constituting the tubular structure 30, or the number of the pores 311 and 312 per unit area of the first layer, may be different from the shape, size, or arrangement of the pores 321 formed in the second layer 320, or the number of the pores 321 per unit area of the second layer. That is, when the shape, size, arrangement of pores or the number of pores per unit area of each layer is set differently for each of the layers, the content of the physiologically active substance in the pores 311 and 312 of the first layer 310 may be different from the content of the physiologically active substance in the pores 321 of the second layer 320.

According to the third embodiment, the first layer 310, which is the innermost layer of the tubular structure 30, may be formed so that the porosity of the pores 311 and 312 is smaller than those of the other layers. In addition, the first layer 310 may be formed so that the number of the pores 311 and 312 is smaller than those in the other layers. Thereby, the first layer 310 may not contain the physiologically active substance at all or may contain the physiologically active substance in a smaller amount than the other layers.

In the third embodiment, the material of each of the layers constituting the tubular structure 30 may be as described above with respect to the first embodiment.

The tubular structure 30 according to the third embodiment may be manufactured by spraying a mixed solution of a polar solvent, a non-polar solvent and a physiologically active substance toward the outer circumferential surface of the third layer 330 in a state in which the first layer 310, the second layer 320 and the third layer 330 are stacked in advance. That is, it may be manufactured by positioning a spraying device outside the tubular structure 30 and spraying the solution toward the outer circumferential surface of the third layer.

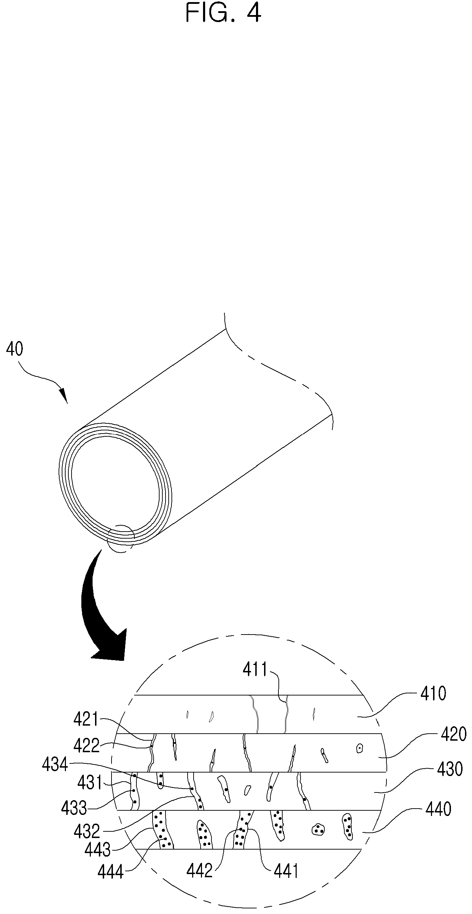

FIG. 4 schematically shows a tubular structure according to a fourth embodiment, according to the present invention. Referring to FIG. 4, the tubular structure 40 according to the fourth embodiment may be formed by stacking four layers. That is, a first layer 410 is the innermost layer of the tubular structure 40. A second layer 420 is attached to the outer circumferential surface of the first layer 410, a third layer 430 is attached to the outer circumferential surface of the second layer 420, and a fourth layer 440 is attached to the outer circumferential surface of the third layer 430. The fourth layer 440 is the outermost layer of the tubular structure 40.

In the fourth embodiment, a plurality of pores may be formed in each of the layers constituting the tubular structure 40. According to the fourth embodiment, a plurality of pores may be formed in the first layer 410, the second layer 420, the third layer 430 and the fourth layer 440. In addition, a physiologically active substance may be contained in at least one pore in at least one of the layers.

In the fourth embodiment, the physiologically active substance may be of a type different from that in the first embodiment. For example, the physiologically active substance according to the fourth embodiment may be an anti-inflammatory agent. In the case in which it is preferable in terms of the efficacy of the physiologically active substance that the physiologically active substance be contained in an outer layer of the tubular structure 40 rather than contained in an inner layer of the tubular structure 40, control may be made so that the physiologically active substance is selectively applied only to the outer layer without existing in the inner layer of the tubular structure 40.

According to the fourth embodiment, the porosity of the pores 411 formed in the first layer 410 among the plurality of layers may be different from the porosity of the pores 421 formed in the second layer 420. That is, by making the porosity of the pores different between the layers, it is possible to control the content of the physiologically active substance in the pores 411 of the first layer 410 so as to be different from the content of the physiologically active substance in the pores 421 of the second layer 420.

As a specific example, in the fourth embodiment, the porosity of the pores 441 and 443 formed in the fourth layer 440 among the four layers is larger than the porosity of the pores 431 and 432 formed in the third layer 430, the porosity of the pores 431 and 432 formed in the third layer 430 may be larger than the porosity of the pores 421 formed in the second layer 420, and the porosity of the pores 421 formed in the second layer 420 may be larger than the porosity of the pores 411 formed in the first layer 410. Thus, the content of the physiologically active substance in the pores of each layer decreases in the direction from the fourth layer 440, which is the outermost layer of the tubular structure 40, toward the first layer 410 which is the innermost layer. In addition, the porosity of the pores may be controlled so that the pores 411 of the first layer 410 do not contain the physiologically active substance, unlike the second layer 420, the third layer 430 and the fourth layer 440.

According to the fourth embodiment, the shape, size, or arrangement of the pores 411 formed in the first layer 410 among the plurality of layers constituting the tubular structure 40, or the number of the pores 411 per unit area of the first layer, may be different from the shape, size, or arrangement of the pores 421 formed in the second layer 420, or the number of the pores 421 per unit area of the second layer. That is, when the shape, size, arrangement of pores or the number of pores per unit area of each layer is set differently for each of the layers, the content of the physiologically active substance in the pores 411 of the first layer 410 may be different from the content of the physiologically active substance in the pores 421 of the second layer 420.

Meanwhile, according to the implementation, the first layer 410, which is the innermost layer of the tubular structure 40, may also contain the physiologically active substance in a smaller amount than the other layers.

According to the fourth embodiment, the first layer 410, which is the innermost layer of the tubular structure 40, may be formed so that the porosity of the pores 411 is smaller than those of the other layers. In addition, the first layer 410 may be formed so that the number of the pores 411 is smaller than those in the other layers. Thereby, the first layer 410 may not contain the physiologically active substance at all or may contain the physiologically active substance in a smaller amount than the other layers.

In the fourth embodiment, the material of each of the layers constituting the tubular structure 40 may be as described above with respect to the first embodiment.

In another embodiment, when the tubular structure is formed of three or more layers, the porosity of a layer positioned next to the outermost layer of the tubular structure may be smaller than the porosities of the other layers. In another embodiment, when the tubular structure is formed of three or more layers, the porosity of a layer positioned next to the outermost layer of the tubular structure may be 0.1 µm to less than 25 µm. Accordingly, the innermost layer or the outermost layer of the tubular structure may not contain the physiologically active substance at all or may contain the physiologically active substance in very small amounts.

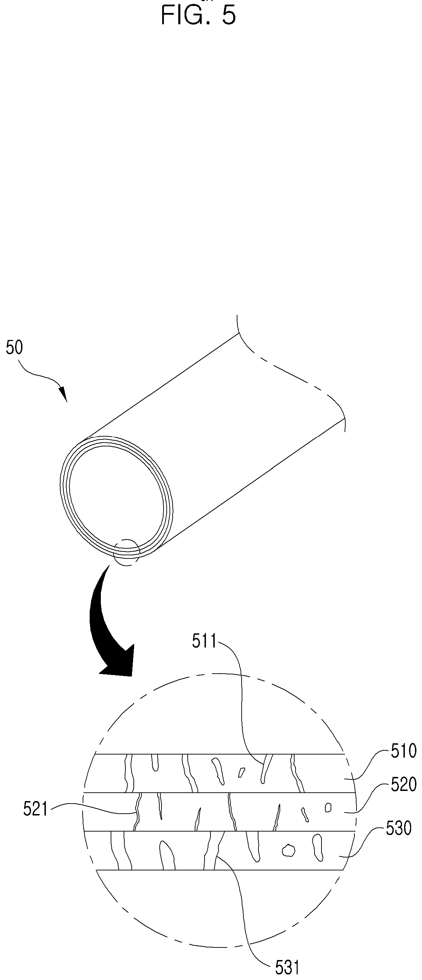

FIG. 5 schematically shows a tubular structure according to a fifth embodiment, according to the present invention. Referring to FIG. 5, the tubular structure 50 according to the fifth embodiment may be formed by stacking three layers.

That is, a first layer 510 is the innermost layer of the tubular structure 50. A second layer 520 is attached to the outer circumferential surface of the first layer 510, and a third layer 530 is attached to the outer circumferential surface of the second layer 520. The third layer 530 is the outermost layer of the tubular structure 50.

In the fifth embodiment, a plurality of pores may be formed in each of the layers constituting the tubular structure 50. According to the fifth embodiment, a plurality of pores may be formed in the first layer 510, the second layer 520, and the third layer 530. In addition, a physiologically active substance may be contained in at least one pore in at least one of the layers. In the fifth embodiment, the physiologically active substance may be of the same or different type as that in the first embodiment.

According to the fifth embodiment, when the tubular structure 50 is formed of three layers, the second layer 520 positioned between the third layer 530, which is the outermost layer of the tubular structure 50, and the first layer 510 which is the innermost layer, may have the smallest porosity.

In the fifth embodiment, the material of each of the layers constituting the tubular structure 50 may be as described above with respect to the first embodiment.

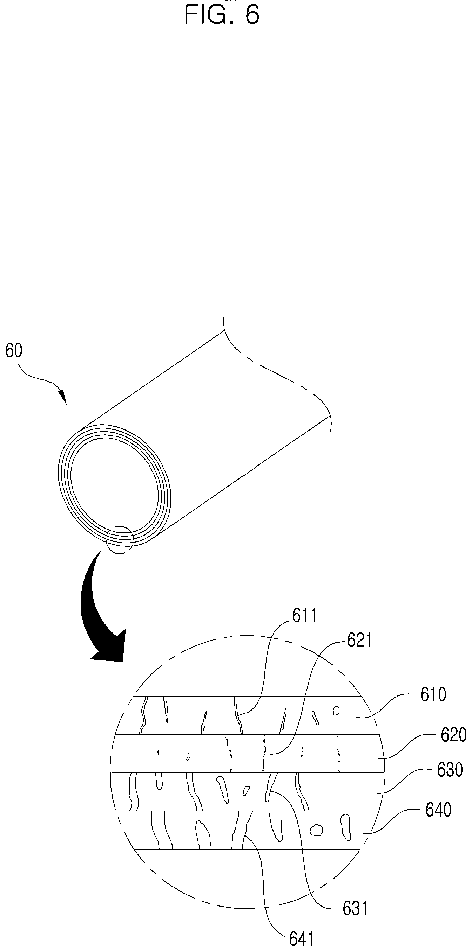

FIG. 6 schematically shows a tubular structure according to a sixth embodiment, according to the present invention. Referring to FIG. 6, the tubular structure 60 according to the sixth embodiment may be formed by stacking four layers.

That is, a first layer 610 is the innermost layer of the tubular structure 60. A second layer 620 is attached to the outer circumferential surface of the first layer 610, a third layer 630 is attached to the outer circumferential surface of the second layer 620, and a fourth layer 640 is attached to the outer circumferential surface of the third layer 630. The fourth layer 640 is the outermost layer of the tubular structure 60.

In the sixth embodiment, a plurality of pores may be formed in each of the layers constituting the tubular structure 60. According to the sixth embodiment, a plurality of pores may be formed in the first layer 610, the second layer 620, the third layer 630, and the fourth layer 640. In addition, a physiologically active substance may be contained in at least one pore in at least one of the layers. In the sixth embodiment, the physiologically active substance may be of the same or different type as that in the first embodiment.

In one embodiment, when the tubular structure is formed of four or more layers, any one of the layers excluding the outermost layer and the innermost layer of the tubular structure may have the smallest porosity.

According to the sixth embodiment, when the tubular structure 60 is formed of four layers, the second layer 620 positioned between the fourth layer 640, which is the outermost layer of the tubular structure 60, and the first layer 610 which is the innermost layer, may have the smallest porosity.

In the sixth embodiment, the material of each of the layers constituting the tubular structure 60 may be as described above with respect to the first embodiment.

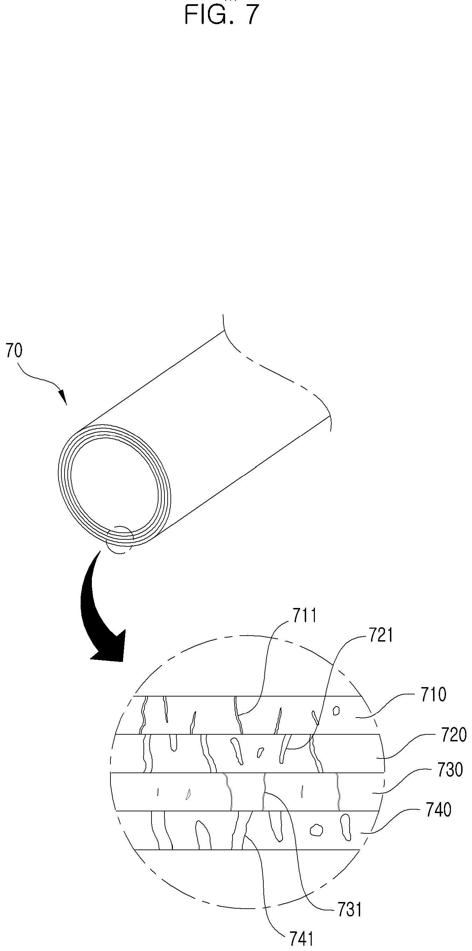

FIG. 7 schematically shows a tubular structure according to a seventh embodiment, according to the present invention. Referring to FIG. 7, the tubular structure 70 according to the seventh embodiment may be formed by stacking four layers.

That is, a first layer 710 is the innermost layer of the tubular structure 70. A second layer 720 is attached to the outer circumferential surface of the first layer 710, a third layer 730 is attached to the outer circumferential surface of the second layer 720, and a fourth layer 740 is attached to the outer circumferential surface of the third layer 730. The fourth layer 740 is the outermost layer of the tubular structure 70.

In the seventh embodiment, a plurality of pores may be formed in each of the layers constituting the tubular structure 70. According to the seventh embodiment, a plurality of pores may be formed in the first layer 710, the second layer 720, the third layer 730, and the fourth layer 740. In addition, a physiologically active substance may be contained in at least one pore in at least one of the layers. In the seventh embodiment, the physiologically active substance may be of the same or different type as that in the first embodiment.

According to the seventh embodiment, when the tubular structure 70 is formed of four layers, the third layer 730 positioned between the fourth layer 740, which is the outermost layer of the tubular structure 70, and the first layer 710 which is the innermost layer, may have the smallest porosity.

In the seventh embodiment, the material of each of the layers constituting the tubular structure 70 may be as described above with respect to the first embodiment.

According to one embodiment, in the case in which any one layer positioned between the outermost layer and the innermost layer of the tubular structure has the smallest porosity, when a syringe needle penetrates the surface of the tubular structure and then the needle is removed, the puncture point of the layer having the smallest porosity may easily shrink, thereby preventing leakage.

According to another embodiment, the outermost layer of the tubular structure may have a larger porosity than any one of the layers excluding the outermost layer and the innermost layer of the tubular structure. In this case, the outermost layer of the tubular structure may maintain a certain porosity, and thus skin tissue may easily penetrate into the pores of the outermost layer, which is in direct contact with the skin, and the tight coupling between the tubular structure and the skin may occur, so that the tubular structure may be stably maintained *in vivo* without departing from the initial placement position.

As described above, according to various embodiments of the present invention, the content of the physiologically active substance may be set differently between the layers, and thus the release rate of the physiologically active substance *in vivo* may be appropriately controlled.

In addition, according to various embodiments of the present invention, as the content of the physiologically active substance in each layer decreases in the direction from the innermost layer to the outermost layer of the tubular structure, and the outermost layer does not contain the physiologically active substance, it is easy for myofibroblasts to proliferate outside of the vascular conduit.

In addition, according to various embodiments of the present invention, as the size, number, shape, arrangement, etc. of pores are set differently for each of the layers, the content of the physiologically active substance in the pores and the release rate thereof may be controlled differently for each of the layers. Thereby, it is possible to prevent a physiologically active substance (e.g., paclitaxel, rapamycin, everolimus, etc.) that inhibit cell proliferation or cell migration from permeating from the inner circumferential surface to the outer circumferential surface of the tubular structure and reaching the outer circumferential surface. In addition, even when a liquid mixture of a physiologically active substance and an organic solvent is applied to the tubular structure, it is possible to prevent the organic solvent from reaching the outer circumferential surface of the tubular structure and deforming or damaging the outer circumferential surface of the tubular structure, thereby preventing unexpected side effects from occurring *in vivo.*

In addition, according to the implementation, the content of the physiologically active substance in each layer may controlled so that it decreases in the direction from the outermost layer of the tubular structure toward to the innermost layer and the innermost layer does not contain the physiologically active substance. Accordingly, in consideration of the efficacy and type of physiologically active substance, control may be made so that a physiologically active material (e.g., an anti-inflammatory agent) is selectively applied only to the outside of the tubular structure.

### [Description of Reference Numerals]

10, 20, 30, 40, 50, 60, 70: tubular structure
110, 210, 310, 410, 510, 610, 710: first layer
111, 112, 211, 212, 213, 311, 312, 411, 511, 611, 711: pores
113, 114, 214, 215, 216: physiologically active substance
120, 220, 320, 420, 520, 620, 720: second layer
121, 221, 321, 421, 521, 621, 721: pores
122, 222, 322, 422: physiologically active substance
130, 230, 330, 430, 530, 630, 730: third layer
131, 132, 231, 232, 331, 332, 431, 432, 531, 631, 731: pores
233, 234, 333, 334, 433, 434: physiologically active substance
240, 440, 640, 740: fourth layer
241, 441, 443, 641, 741: pores
442, 444: physiologically active substance

## Claims

1. A porous multi-layered tubular structure containing a physiologically active substance in pores, the tubular structure comprising a tubular structure in which a plurality of layers are stacked,
wherein a plurality of pores are formed in each of the layers, and the physiologically active substance is contained in the pores of at least one of the layers,
**characterized in that**,
each of the layers is made of at least one material selected from the group consisting of expanded polytetrafluoroethylene, polytetrafluoroethylene, and polyurethane,
the tubular structure is formed of three or more layers, and a porosity of a layer positioned next to an outermost layer of the tubular structure is smaller than porosities of the other layers, as measured according to International Standardization Organization (ISO) standard 7198:1998.

2. The porous multi-layered tubular structure according to claim 1, wherein a content of the physiologically active substance in at least one of the layers is different from a content of the physiologically active substance in the other layer.

3. The porous multi-layered tubular structure according to claim 1, wherein at least one of the layers is formed to be different from the other layer in terms of a shape of the pore, a size of the pore, an arrangement of the pores, or a number of the pores per unit area of the layer, so that a content of the physiologically active substance in the pores of each of the layers is set differently for each of the layers.

4. The porous multi-layered tubular structure according to claim 1, wherein the physiologically active substance is a substance that inhibits cell proliferation or cell migration.

5. The porous multi-layered tubular structure according to claim 1, wherein the physiologically active substance is at least one selected from the group consisting of paclitaxel, rapamycin, and everolimus.

6. The porous multi-layered tubular structure according to claim 1, wherein a content of the physiologically active substance in each of the layers decreases in a direction from an innermost layer to an outermost layer of the tubular structure.

7. The porous multi-layered tubular structure according to claim 6, wherein the outermost layer of the tubular structure does not contain the physiologically active substance.

8. The porous multi-layered tubular structure according to claim 1, wherein, when the physiologically active substance is paclitaxel, a content of the physiologically active substance per area of an innermost layer of the tubular structure is 0.25 to 1.5 µg/mm².

9. The porous multi-layered tubular structure according to claim 1, wherein, when the physiologically active substance is paclitaxel, a content of the physiologically active substance per area of a layer positioned next to an innermost layer of the tubular structure is 0.25 to 0.75 µg/mm².

10. The porous multi-layered tubular structure according to claim 1, wherein an outermost layer of the tubular structure does not contain the physiologically active substance, or when the physiologically active substance is paclitaxel, a content of the physiologically active substance per area of the outermost layer is 0.25 µg/mm². or less.

11. The porous multi-layered tubular structure according to claim 1, wherein an outermost layer of the tubular structure has a porosity of less than 25 µm as measured according to International Standardization Organization (ISO) standard 7198:1998.

12. The porous multi-layered tubular structure according to claim 1, wherein a content of the physiologically active substance in each of the layers decreases in a direction from an outermost layer to an innermost layer of the tubular structure.

13. The porous multi-layered tubular structure according to claim 1, wherein, a porosity of a layer positioned next to an outermost layer of the tubular structure is less than 25 µm as measured according to International Standardization Organization (ISO) standard 7198:1998.

14. The porous multi-layered tubular structure according to claim 1, wherein the plurality of layers are all made of the same material

15. The porous multi-layered tubular structure according to claim 1, wherein, when the tubular structure is formed of three layers, a porosity of a layer positioned between an outermost layer and an innermost layer of the tubular structure is smaller than porosities of the other layers.

## Patentansprüche

1. Poröse mehrschichtige tubuläre Struktur, die einen physiologischen Wirkstoff in Poren enthält, wobei die tubuläre Struktur eine tubuläre Struktur umfasst, bei der eine Vielzahl von Schichten aufeinandergestapelt sind,
wobei in jeder der Schichten eine Vielzahl von Poren ausgebildet sind, und der physiologische Wirkstoff in den Poren wenigstens einer der Schichten enthalten ist,
**dadurch gekennzeichnet, dass**
jede der Schichten aus wenigstens einem Material besteht, das aus der Gruppe ausgewählt ist, die aus expandiertem Polytetrafluorethylen, Polytetrafluorethylen und Polyurethan besteht,
wobei die tubuläre Struktur aus drei oder mehr Schichten gebildet ist und die Porosität einer Schicht, die direkt neben einer äußersten Schicht der tubulären Struktur liegt, geringer ist als die Porositäten der anderen Schichten, wenn gemäß der Norm 7198:1998 der International Standardization Organization (ISO) gemessen wird.

2. Poröse mehrschichtige tubuläre Struktur gemäß Anspruch 1, wobei sich der Gehalt des physiologischen Wirkstoffs in wenigstens einer der Schichten von einem Gehalt des physiologischen Wirkstoffs in der anderen Schicht unterscheidet.

3. Poröse mehrschichtige tubuläre Struktur gemäß Anspruch 1, wobei wenigstens eine der Schichten so ausgebildet ist, dass sie sich in Bezug auf die Form der Poren, die Größe der Poren, die Anordnung der Poren oder die Anzahl der Poren pro Flächeneinheit der Schicht von der anderen Schicht unterscheidet, so dass der Gehalt des physiologischen Wirkstoffs in den Poren jeder der Schichten für jede der Schichten unterschiedlich eingestellt ist.

4. Poröse mehrschichtige tubuläre Struktur gemäß Anspruch 1, wobei der physiologische Wirkstoff eine Substanz ist, die die Zellvermehrung oder Zellmigration hemmt.

5. Poröse mehrschichtige tubuläre Struktur gemäß Anspruch 1, wobei der physiologische Wirkstoff wenigstens einer ist, der aus der Gruppe ausgewählt ist, die aus Paclitaxel, Rapamycin und Everolimus besteht.

6. Poröse mehrschichtige tubuläre Struktur gemäß Anspruch 1, wobei der Gehalt des physiologisch aktiven Wirkstoffs in jeder der Schichten in einer Richtung von einer innersten Schicht zu einer äußersten Schicht der tubulären Struktur hin abnimmt.

7. Poröse mehrschichtige tubuläre Struktur gemäß Anspruch 6, wobei die äußerste Schicht der tubulären Struktur den physiologischen Wirkstoff nicht enthält.

8. Poröse mehrschichtige tubuläre Struktur gemäß Anspruch 1, wobei dann, wenn es sich bei dem physiologisch aktiven Wirkstoff um Paclitaxel handelt, der Gehalt des physiologischen Wirkstoffs pro Fläche einer innersten Schicht der tubulären Struktur 0,25 bis 1,5 µg/mm². beträgt.

9. Poröse mehrschichtige tubuläre Struktur gemäß Anspruch 1, wobei dann, wenn es sich bei dem physiologisch aktiven Wirkstoff um Paclitaxel handelt, der Gehalt des physiologischen Wirkstoffs pro Fläche einer Schicht, die direkt neben einer innersten Schicht der tubulären Struktur liegt, 0,25 bis 0,75 µg/mm². beträgt.

10. Poröse mehrschichtige tubuläre Struktur gemäß Anspruch 1, wobei die äußerste Schicht der tubulären Struktur den physiologisch aktiven Wirkstoff nicht enthält oder dann, wenn es sich bei dem physiologisch aktiven Wirkstoff um Paclitaxel handelt, der Gehalt des physiologisch aktiven Wirkstoffs pro Fläche der äußersten Schicht 0,25 µg/mm². oder weniger beträgt.

11. Poröse mehrschichtige tubuläre Struktur gemäß Anspruch 1, wobei eine äußerste Schicht der tubulären Struktur eine Porosität von weniger als 25 µm aufweist, wenn gemäß der Norm 7198:1998 der International Standardization Organization (ISO) gemessen wird.

12. Poröse mehrschichtige tubuläre Struktur gemäß Anspruch 1, wobei der Gehalt des physiologisch aktiven Wirkstoffs in jeder der Schichten in einer Richtung von einer äußersten Schicht zu einer innersten Schicht der tubulären Struktur hin abnimmt.

13. Poröse mehrschichtige tubuläre Struktur gemäß Anspruch 1, wobei die Porosität einer Schicht, die direkt neben einer äußersten Schicht der tubulären Struktur liegt, kleiner als 25 µm ist, wenn gemäß der Norm 7198:1998 der International Standardization Organization (ISO) gemessen wird.

14. Poröse mehrschichtige tubuläre Struktur gemäß Anspruch 1, wobei die Vielzahl der Schichten alle aus demselben Material bestehen.

15. Poröse mehrschichtige tubuläre Struktur gemäß Anspruch 1, wobei dann, wenn die tubuläre Struktur aus drei Schichten gebildet ist, die Porosität einer Schicht, die sich zwischen einer äußersten Schicht und einer innersten Schicht der tubulären Struktur befindet, geringer ist als die Porositäten der anderen Schichten.

## Revendications

1. Structure tubulaire multicouche poreuse, contenant une substance physiologiquement active dans des pores, la structure tubulaire comprenant une structure tubulaire dans laquelle une pluralité de couches sont empilées,
dans laquelle une pluralité de pores sont formés dans chacune des couches, et la substance physiologiquement active est contenue dans les pores d'au moins une des couches,
**caractérisée en ce que**
chacune des couches est constituée d'au moins un matériau choisi dans le groupe consistant en polytétrafluoroéthylène expansé, polytétrafluoroéthylène et polyuréthane,
la structure tubulaire est formée de trois couches ou plus, et la porosité d'une couche positionnée à côté d'une couche la plus externe de la structure tubulaire est plus petite que les porosités des autres couches, telle que mesurée selon la norme 7198:1998 de l'Organisation internationale de normalisation (ISO).

2. Structure tubulaire multicouche poreuse selon la revendication 1, dans laquelle une teneur en substance physiologiquement active dans au moins une des couches est différente d'une teneur en substance physiologiquement active dans l'autre couche.

3. Structure tubulaire multicouche poreuse selon la revendication 1, dans laquelle au moins une des couches est formée pour être différente de l'autre couche en termes de forme du pore, de taille du pore, de disposition des pores ou de nombre de pores par unité de surface de la couche, de sorte qu'une teneur en substance physiologiquement active dans les pores de chacune des couches est fixée différemment pour chacune des couches.

4. Structure tubulaire multicouche poreuse selon la revendication 1, dans laquelle la substance physiologiquement active est une substance qui inhibe la prolifération cellulaire ou la migration cellulaire.

5. Structure tubulaire multicouche poreuse selon la revendication 1, dans laquelle la substance physiologiquement active est au moins une choisie dans le groupe consistant en paclitaxel, rapamycine et évérolimus.

6. Structure tubulaire multicouche poreuse selon la revendication 1, dans laquelle la teneur en substance physiologiquement active dans chacune des couches diminue dans une direction allant d'une couche la plus interne à une couche la plus externe de la structure tubulaire.

7. Structure tubulaire multicouche poreuse selon la revendication 6, dans laquelle la couche la plus externe de la structure tubulaire ne contient pas de substance physiologiquement active.

8. Structure tubulaire multicouche poreuse selon la revendication 1, dans laquelle, lorsque la substance physiologiquement active est le paclitaxel, la teneur en substance physiologiquement active par surface de la couche la plus interne de la structure tubulaire est de 0,25 à 1,5 µg/mm².

9. Structure tubulaire multicouche poreuse selon la revendication 1, dans laquelle, lorsque la substance physiologiquement active est le paclitaxel, la teneur en substance physiologiquement active par surface d'une couche positionnée à côté d'une couche la plus interne de la structure tubulaire est de 0,25 à 0,75 µg/mm².

10. Structure tubulaire multicouche poreuse selon la revendication 1, dans laquelle la couche la plus externe de la structure tubulaire ne contient pas de substance physiologiquement active, ou lorsque la substance physiologiquement active est le paclitaxel, la teneur en substance physiologiquement active par surface de la couche la plus externe est 0,25 µg/mm². ou moins.

11. Structure tubulaire multicouche poreuse selon la revendication 1, dans laquelle une couche externe de la structure tubulaire a une porosité inférieure à 25 µm, telle que mesurée selon la norme 7198:1998 de l'Organisation internationale de normalisation (ISO).

12. Structure tubulaire multicouche poreuse selon la revendication 1, dans laquelle la teneur en substance physiologiquement active dans chacune des couches diminue dans une direction allant d'une couche la plus externe à une couche la plus interne de la structure tubulaire.

13. Structure tubulaire multicouche poreuse selon la revendication 1, dans laquelle, la porosité d'une couche positionnée à côté d'une couche la plus externe de la structure tubulaire est inférieure à 25 µm, telle que mesurée selon la norme 7198:1998 de l'Organisation internationale de normalisation (ISO).

14. Structure tubulaire multicouche poreuse selon la revendication 1, dans laquelle la pluralité des couches sont toutes constituées du même matériau.

15. Structure tubulaire multicouche poreuse selon la revendication 1, dans laquelle, lorsque la structure tubulaire est formée de trois couches, la porosité d'une couche positionnée entre une couche la plus externe et une couche la plus interne de la structure tubulaire est plus petite que les porosités des autres couches.
